# EUROPEAN PATENT APPLICATION

(11) **EP 4 354 454 A1**
(43) Date of publication of application: **17.04.2024**
(21) Application number: 22306555.8
(22) Date of filing: 13.10.2022
(51) Int. Cl.: G16H 40/63, G16H 50/20, G16H 80/00, G06F 3/01, A61N 5/06, G02B 27/01

(54) **VIRTUAL REALITY DEVICE FOR NEUROLOGICAL DISEASES TREATMENT**

(71) Applicant: Clarity Technologies, 75002 Paris (FR)
(72) Inventor: CERTAIN, Raphael, 31620 LABASTIDE ST SERNIN (FR)
(74) Representative: Flesselles, Bruno F.G.

(57) **Abstract**

The invention relates to a system for displaying virtual reality scenarios and sensory stimulation protocols to patients with neurological diseases, in particular Alzheimer's disease.

## Description

The present invention relates to the general technical field of medical devices, and in particular to monitoring, caregiving or treating patients with neurological diseases, in particular Alzheimer's disease.

With 90 million deaths and 16% of global annual deaths, neurological disorders are the second leading cause of death after heart disease and the leading cause of disability. Neurological disorders include Stroke, spine injuries, Epilepsia, sleep disorders, Alzheimer's Disease, Parkinson's Disease, Dementia with Lewy Bodies, Primary Progressive Aphasias (PPA), Frontotemporal Dementia, Corticobasal Syndrome, Progressive Supranuclear Palsy, and Posterior Cortical Atrophy, and others. In these diseases, the cognitive ability of the patients is impaired and may also decline overtime.

With the aging population, the number of people with neurological disorders will increase and be a heavy burden on society and healthcare systems.

With more than 46.8 million people worldwide, dementia is one of the most devastating neurological disorders. The number of patients is expected to increase to 74.7 million people by 2030, and to 131.5 million people by 2050. More than 60% of dementia cases are caused by Alzheimer's disease (AD). Among the neuropathophysiological features associated with AD, one can generally observe an increase in amyloid-beta protein (AB), hyperphosphorylation of tau protein, inflammatory activity of microglia, and dendritic tree degeneration. It has also been shown that neural oscillations, particularly theta-gamma coupling, are disrupted in AD and that these impairments manifest before any behavioral deficit.

AD induces spatial, motor and cognitive impairments, especially in memory and reasoning, and not effective treatments are currently available. Over the last few years, research on AD has begun to examine the potential of non-pharmacological techniques to influence the symptoms of the disease and define new possible therapeutic opportunities. In particular, stimulation of the patients would slow down the evolution of the disease.

As AD is also one of the neurological disorders implying the most important assistance needs, as the disease progresses toward less self-sufficiency, there is a need to slow down the evolution of the disease, to maintain some kind of independence of the patient as long as possible.

There is also a need to provide tools to the AD patients, allowing them to be stimulated, in an autonomous way, while guaranteeing their safety and the safety of others. Preferably, and as AD can be considered as locking the patients down within themselves, the tools would also preferably help inducing interactions of the patient with the environment.

The invention thus provides a device for brain stimulation of patients with neurological disorders, based on virtual reality (VR) systems (thereby making possible to encourage the patients to interact with the environment, although in the virtual reality environment), and providing sensory stimulations, to act on the patients" brain. In some embodiments, these devices can also be used for monitoring health of the patients, by measuring various parameters associated with physiological activity. This can also allow determining the effect of the sensory stimulations on the patients and adjusting or adapting such stimulations, and can, under some conditions, allow monitoring evolution of the disease.

A few noninvasive brain stimulation (NIBS) methods, providing the ability to modulate neural activity without penetrating the skin, have shown to be promising alternatives to pharmacological treatments. One can cite electrical stimulation like transcranial direct current stimulations (tDCS) or transcranial alternating current stimulations (tACS), magnetic stimulations like transcranial magnetic stimulation (TMS), and multisensory stimulations like audio-visual stimulations (AVS). Other invasive methods are well known, studied and use like deep brain stimulation (DBS) or optogenetics.

Those methods allow triggering the activity of specific brain regions, neural populations, and brain waves in order to modulate cognitive and motor functions.

tACS, tDCS, TMS and AVS have all shown to provide benefits against cognitive decline and neurodegenerative diseases, among which Alzheimer's Disease, Parkinson's Disease, Dementia with Lewy Bodies, Primary Progressive Aphasias (PPA), Frontotemporal Dementia, Corticobasal Syndrome, Progressive Supranuclear Palsy, and Posterior Cortical Atrophy. Those methods have also shown to be particularly promising for accelerating recovery after brain injuries like stroke. NIBS are also methods allowing identification of neural-based biomarkers to deliver diagnoses and assess the cognitive and motor abilities of patients.

The central nervous system (CNS) is modulated by chemical and electrical activity. Neurons, as part of the CNS can fire at frequencies ranging from ~1Hz to -120Hz. Frequency bands are classified from the lower frequency to the highest frequency and named delta, theta, alpha, beta and gamma. They are reported to be associated with and underlying specific cognitive and motor functions. Gamma brain waves' frequency range is known to be beyond 30Hz. Gamma bands have been shown to be involved in higher cognitive abilities including decision-making, reasoning and memory. Studies have demonstrated that Alzheimer's patients have abnormal gamma activities compared to healthy people of the same age category. NIBS aiming at training gamma activity is a promising approach to treating Alzheimer's disease and related pathologies.

A few studies have shown that gamma entrainment using sensory stimulus (visual or auditory at 40 Hz) in mouse models has beneficial effects on neuropathophysiological features of Alzheimer's disease. In particular, they have indicated improvement in cognitive functions. The simultaneous delivery of visual and auditory gamma stimuli increases the effects more than a single stimulation with either modality alone. Recent findings also showed that this neurostimulation technology is safe and offers improvement of AD-related-degeneration biomarkers as well as improving sleep quality.

It has also been shown that sensory stimulation (either visual, auditory, or both) could enhance human neural oscillations in the theta frequency band and enhance memory performance in tasks probing e.g. associative memory.

It is of interest for patients who are still autonomous (with Low or Mild Cognitive Impairments (MCI)) to have these kinds of sensory stimulation on a regular basis (preferably one hour per day) to slow down the evolution of the disease.

Virtual reality can be defined as a computer-generated simulation of a three-dimensional image or environment that can be interacted with in a seemingly real or physical way by a person using special electronic equipment, such as a helmet with a screen inside and/or gloves fitted with sensors. It thus allows the user to navigate in a digital environment, interact with the environment, and carry out specific tasks.

US20180280656 describes integrated systems for providing audio, video, light stimulus and/or measuring brain activity through the use of one or more electrodes, e.g., EEG dermatrodes, are described. A system, e.g., a head mounted VR device, for providing stimulation to a user and detecting user responses to said stimulation includes a head mount, a plurality of light emitting elements, e.g., sets of LEDs, a housing secured to said head mount, and at least one or more electrodes for making contact with skin of a user. In various embodiments, the system further determines a user's mental state and provides audio and/or visual feedback to the user. The system described in this document presents plurality of light emitting elements (40 or 42) which are secured are secured around left eye optics 44 and right eye optics 46.

In a first embodiment, the invention pertains to a system for brain stimulation, comprising:
(a) at least one virtual reality system comprising a virtual reality headset, sensors and apparatus for continuously measuring in real time at least one physiological parameter of the patient, wherein the virtual reality headset comprising a screen
(b) at least one control hardware in which a first library of sensory stimulations protocols is loaded, and a second library of virtual reality scenarios [adaptative scenarios] is loaded
(c) at least one calculation and analysis algorithm loaded on the control hardware to determine the nature of the physiological parameter measured by the sensors apparatus devices, and to control the operation of the virtual reality system according to the nature of the measured physiological parameter by :
   i) selecting in real time from the first library a sensory stimulation protocol or a succession of sensory stimulation protocols, according to the nature of the measured physiological parameter, wherein the sensory stimulation protocol or succession of sensory stimulation protocols comprise visual sensory stimulation elements
   ii) optionally selecting in real time from the second library a virtual reality scenario or a succession of virtual reality scenarios, according to the nature of the measured physiological parameter; and
   iii) adapting or modulating in real time the sensory stimulation protocols and the virtual reality scenarios selected as a function of the evolution of the measured physiological parameter (in particular brain activity) to provide the patient sensory stimulations able to stimulate the brain activity of the patient.
wherein the system is configured to broadcast the selected virtual reality scenarios selection and the visual sensory stimulation elements in the virtual reality headset (i.e. on the screen of the virtual reality headset). As indicated below, the sensory stimulations are adapted or modulated in iii) to have them personalized for the patient during the session.

The system and device may also include sensors to detect physiological parameters of the patient, and thus be used for monitoring the patient's health and/or evolution of the neurological disorder.

The display of the visual sensory stimulation elements (or events) is performed on the screen of the virtual reality headset, which ensures that the patient will properly receive these stimulation elements. This differs from the device proposed in US20180280656, where the visual stimuli are presented by LEDs surrounding right and left eye lenses, and are thus spatially fixed. In contrast, using the screen to display the visual stimuli allows choosing where the stimuli is presented in front of the patient's eyes. Variation of the localization of the visual stimuli during the session may provide benefits to the patient; in particular when a given pattern of stimuli is presented, the patient may anticipate location of the following stimulus, which is of benefit for his brain activity. The fact that the visual stimuli are presented on the VR screen makes it possible to be able to directly modulate the rhythmic visual stimulus of the screen as desired and provide a more pleasant and immersive experience for the patient. This would increase adherence of the patients with the protocol in the long term. The sensory stimulations can also be combined with the VR environment, which may thus increase the therapeutic potential. Indeed, using the VR screen to present visual stimuli makes it possible to more efficiently mobilize the visual spectrum, as there is a better perception of the stimuli when the stimuli are presented in the axis of the visual field and not around it, as described in US20180280656. This would lead to a better induced neural training and hence to better therapeutic benefits.

It is preferred when the stimulations provided to the patient are rhythmic stimulations (*i.e.* are presented on a regular rhythm, at given frequencies (as opposed, for example, to random stimulations). In view of the preferred frequency of the stimulating lights (flash of lights), such provision of the visual stimulation on the VR screen requires that the screen presents a refresh rate high enough to be adapted to the frequency of the light flashes. A refresh rate of 120 Hz or higher is particularly advantageous. Such refresh is very unusual, as the regular TV screens or monitors have a refresh rate that is too low to run visual stimulations between 30-60 Hz and in particular around 40 Hz. Since current Virtual Reality headsets are generally used for presenting Virtual Reality scenarios (such as gaming or teaching) without the need to provide sensory stimulation at high frequencies, they would not present such ability to do so at these frequencies.

The device herein disclosed allows performing methods comprising:
a) continuously measuring in real time at least one physiological parameter of the patient,
b) using at least one calculation and analysis algorithm to determine, from the values measured in a), one sensory stimulation protocol or a succession of sensory stimulation protocols to provide to the patient, and selecting the sensory stimulation protocol or a succession of sensory stimulation protocols, wherein the sensory stimulation protocol or succession of sensory stimulation protocols comprises a visual stimulation protocol
c) optionally selecting in real time from a library of virtual reality scenarios, a virtual reality scenario or a succession of virtual reality scenarios, depending on the value measured in a) or depending on the sensory stimulation protocol or a succession of sensory stimulation protocols selected in b)
d) broadcasting the virtual reality scenario(s) of c) in a virtual reality system headset worn by the patient, and applying the selected simulation protocol(s) of b), wherein the virtual reality scenario and the visual stimulation protocol are displayed on the screen of the headset,
e) adapting or modulating in real time the selection made under b) and/or c) according to the evolution of measure of the physiological parameter of the patient.

The device thus makes it possible to provide personalized settings, in real time (in particular by adjusting frequencies of the stimuli), to induce optimized neural training to the patient.

The method thus uses at least one feedback loop, using the information on the patient's physiological state, to adapt in real time the stimulation protocols, and optionally the virtual reality scenario. The virtual reality scenario can be adapted depending on the response of the patient as determined by the measured physiological parameters, or to be adapted to the new sensory stimulation selected protocol.

The physiological parameters measured may be selected from signals from EEG (electroencephalogram), heart rate, pupilometry, eye movement, electrodermal conductance, body temperature, respiratory rate, heart rate variability (HRV).

Any other physiological parameter that can be collected non-invasively and that provides information on the patient's physiological state in real time may be collected using appropriate sensors.

In a preferred embodiment, the physiological parameter of the patient measured in a) comprises brain activity and the sensors comprise a ElectroEncephaloGraphy headset (EEG). The brain activity can be detected using electrodes, which measure electrical activity that can be processed and so as to determine which portions of a person's brain is active (depending on the area where the electrode is placed on the patient's skin) and from this the user's mental state. It is of particular interest to measure the EEG signal, and in particular the phase and/or the amplitude of the theta brain waves (if a 4Hz stimulation is provided) or of the gamma brain waves (if a 40Hz stimulation is provided) or of both brain waves. The sensory stimulation protocol is then selected from the library to provide at least visual stimulation that are in phase with the actual phase measured from the patient. This would optimize the therapeutic effect. In other words, one can also say that the selection of the stimulatory protocol depending on the measured physiological parameters corresponds to adapting the stimulation protocols depending on the measured physiological parameters.

In addition, it is of great interest to measure at least one of heart rate, eye movement, pupilometry and body temperature.

In one embodiment, the device and system may also be designed so that the virtual reality headset comprises integrated audio speakers for auditory stimuli. Such headsets can be used to provide sounds in relation with the virtual reality scenario (such as instructions, questions or the like) to interact with the patient, and make the patient act in the virtual reality universe. They can also be used to provide sound stimuli to the patient. An embodiment where such sound stimuli are also provided (together with the visual stimuli) is preferred.

It is also preferred when the auditory (sound) stimulations are phase-locked to the visual stimulations. It is sought to obtain such synchronization between the sound and visual stimuli, and keep the two stimuli phase-locked overtime, including when the frequency of the stimuli vary depending on the phase of the theta and/or gamma brain waves, as disclosed above. It enables a perfect synchronization of auditory and visual stimuli, a phase-locking between stimulation frequencies (if multiple ones are combined e.g 4-40Hz coupling), and a phase-locking of stimulation frequencies with corresponding endogenous frequencies (brain waves). Obtaining a proper synchronization makes it possible to improve the therapeutic effects (such as cognition) while an improper synchronization is susceptible to induce negative effects.

In some embodiments, the virtual reality headset system further comprises an electrical stimulator and electrodes that can be used to provide peripheral or deep brain nerve stimuli to the patient.

Electrodes for peripheral nerve stimulation shall be placed on the surface of the skin. The main nerves that can be stimulated are the vagus nerve in the neck, the median nerve and/or the radial nerves.

Electrodes for deep brain stimulation are generally implanted in the motor cortex, before performance of the method. The virtual reality headset shall thus comprise connectors for being plugged to the implanted electrodes. Such deep brain stimulations are of interest in particular for Parkinson's disease.

In both cases, the stimulations are therefore electrical. They are preferably rhythmic, synchronized with the frequency of audiovisual stimulations delivered in the virtual reality headset. In other embodiments, it is foreseen to that the electric stimulations are delivered in synchronization with the virtual reality scenario and delivered to assist the patient when required to perform some task in the virtual reality environment.

The second library (digital library or database) comprises virtual reality scenarios. The virtual reality scenarios can comprise immersion in a 3D virtual visual universe, a specific sound environment, notably music, animated images. It is preferred when the scenarios of the second library comprise reproduction of places and spaces, potentially known by the patient, in order to allow the patient to practice relaxation exercises or improve cognitive abilities and interaction of the patient in the virtual reality universe. Some virtual reality scenarios may also include cognitive games to improve cognitive (such as memory, attention, sleep) and/or functional (such as motor) abilities of the patient.

The first library comprises protocols to provide stimulations to the patient. Various type of stimulations can be applied. One can cite light simulations (in particular at specific frequencies (preferably comprised between 1Hz and 100 Hz, preferably at 4 Hz and/or 40 Hz), sound stimuli (preferably phase locked with the visual stimuli), or electric stimuli, in particular to the peripheral nerves. This is of particular interest when coupled with virtual reality scenarios that are designed for requiring movements of the patients, to improve his motor ability. Such kinds of scenario are of interest for instance for the recovery of stroke, or for treating patients with Parkinson's disease. In particular, it is preferred to start the session with light simulations at 4 Hz, 40 Hz or both, and to adapt in real time the frequency of these light stimulation to have them become in phase with the frequency of the brain waves as measured by the EEG. The new frequencies of the light stimuli would not change dramatically, but would be around the starting frequencies (for instance between 3.8 and 4.2 Hz of 38-42 Hz). The frequencies of theta and gamma brain waves of people are around 4-8 Hz and 30-100 Hz respectively. However, since the frequency having shown to be involved in disrupted cognitive functions in Alzheimer's are 4Hz and 40Hz and the coupling of 4Hz-40Hz in the brain is generally disrupted in AD patients and is in part responsible of poor memory abilities, it is of interest to stimulate at these frequencies. In preferred embodiments, sound stimuli, phase-locked or synchronized with the visual stimuli are also provided during the session. In this case, it is preferred when the sound stimuli are applied together with the visual stimuli from the start of the session.

In preferred embodiments, the system also comprises a user interface for selecting an initial virtual reality scenario or sequence of virtual reality scenarios, and a stimulation protocol. The scenarios and protocols shall change during the session, depending on the responses of the patient to the stimulation (as measured by the sensors of the system) and of its interactions in the virtual reality interface, and according to algorithms in the system for optimizing the stimulation parameters and/or virtual reality scenario and selecting the optimized items from the first and second libraries.

The system may also include a database in which are stored, for the patients, the virtual reality scenario and stimulation protocols applied, the response of the patient to these virtual reality scenario and stimulations, including physiological activity measurements, health scores, and brain activity recordings during the stimulation protocols. Such database may be accessed to by the user interface of the system, and/or remotely by medical teams people to help them personalize the therapeutic protocols, and in particular the nature of the initial stimulations that would be applied for the next session for the patient.

The system thus shall work in closed-loop, where the physiological parameters shall be monitored and recorded, and their value or variation is used to adapt the stimulation protocols and/or virtual reality scenarios.

In some embodiments, the system also comprises at least one server on which the databases (libraries) relating to virtual reality scenarios and stimulation protocols are loaded. The server is connected to control equipment via a wired or wireless communication link, preferably by encrypted transmission. The system in accordance with the invention can thus be integrated into a communication network, for example wifi, 4G, 5G or others. It is foreseen to provide updates of the system via updates of libraries of the server.

In some embodiments, the system may further comprise a software module for transmitting (most preferably using encrypted transmission) the physiological parameter data as recorded by the sensors to a remote server or database/library *via* a computer network. In particular, an ID associated to the patient and/or a time stamp and/or the type of stimulation protocols and virtual reality scenarios can be transmitted at the same time. The patient's physician may thus easily access this data, and follow-up the evolution of the clinical condition of the patient.

The various protocols for selecting, displaying the virtual reality scenarios and stimulation protocols, or analyzing the physiological parameters received from the sensors can be performed by means of computer programs comprising program code instructions for performing the steps of the method herein disclosed, when said program is run on a computer.

The invention also relates to an *ex vivo* computer-implemented method for operating a system herein disclosed, comprising
(a) receiving the information related to the initial stimulation protocol and virtual reality scenario selected by an operator on the user interface
(b) receiving data relating to the physiological and neural activity from the sensors (such data is received in real-time from the measurements made by the sensors)
(c) determining modifications to apply to the virtual reality environment (scenario) and/or stimulations, on the basis of the data received in (b), so as to adapt and optimize such elements; such optimization / adaptation may include modifying the shape, the onset, the frequency, the intensity (lux, color, sound, volume...) of the stimulations to provide to the patient;
(d) sending instructions to a processor for selecting virtual reality environment (scenario) and stimulations to be displayed to the headset in the first and second libraries.

In some embodiments, steps (c) and (d) also comprise stopping the stimulation as VR scenario if abnormal neural activity patterns (in particular epileptic patterns) are identified from the received physiological parameters that were measured by the sensors. This thus encompasses a method to detect any adverse event due to the stimulations (such as triggering seizure) and instantaneously stopping the stimulations in order to prevent its or degradation of the health of the patient.

It is reminded that an epileptic seizure is a temporary event of symptoms due to synchronization of abnormally excessive activities of neurons in the brain. "Epileptiform abnormalities" or "epilepsy waves" can be detected on an EEG. They can look like spikes, sharp waves, and spike-and-wave discharges. The seizure, on the EEG recording, will lead to the appearance of abnormal discharges in bursts, which are called ictal epileptiform discharges. These discharges increase in frequency and lead to rapid continuous spikes and waves, that progress to numerous spikes with buried waves (at the peak of the seizure activity). Eventually, the waves will reappear and progressively reduce in frequency when the seizure calms down. Detection of the epileptic seizure can be performed by the analysis and calculation module, for instance by comparing the received EEG data to a library containing representative patterns of waves during epileptic events, or by detecting a change of the EEG wave, as disclosed above (detection of the ictal epileptiform discharges and increase in frequency thereof). Machine learning algorithms, trained on EEG of patients with epileptic crisis (especially on patterns at the start of the crisis) and patients without such crisis, can also be used to infer and detect the start of an epilepsy event, and thus be able to stop the session (in particular the visual stimulations).

Generally speaking, the method shall include:
- analysis of brain oscillations during visual stimulation at a given frequency in the headset, from the EEG recording sent to the analysis and calculation module
- quantification of the predominant brain frequencies
- analysis of the amplitude and phase of the brain oscillations
- adapting the onset and frequency of the stimuli (visual and optionally sound stimulations, while maintaining phase synchronization between visual and sound stimulations) for synchronization with the phase and amplitude of the target brain oscillation. This corresponding to the step of selecting the stimulations to be displayed to the headset.
- Optionally modifying the VR scenario (corresponding to selecting another VR scenario), for instance by adapting the visual stimuli (such as the shape of the object presenting the rhythmic stimuli, the color of the object, the frequency of stimuli ...) that are incorporated in the VR environment, and/or modulation of audiovisual stimuli presented in the VR environment for synchronization with brain oscillations as described above.
- sending instructions for displaying the novel stimulations through the headset.

In some embodiments, the method also comprise calculating cognitive functions scores (such as sleep, memory, attention, motor abilities and others), disease risk score or brain health score, using the parameters received from the sensors. Such information and scores may be sent to the remote server mentioned above to be made available to the patient's physician.

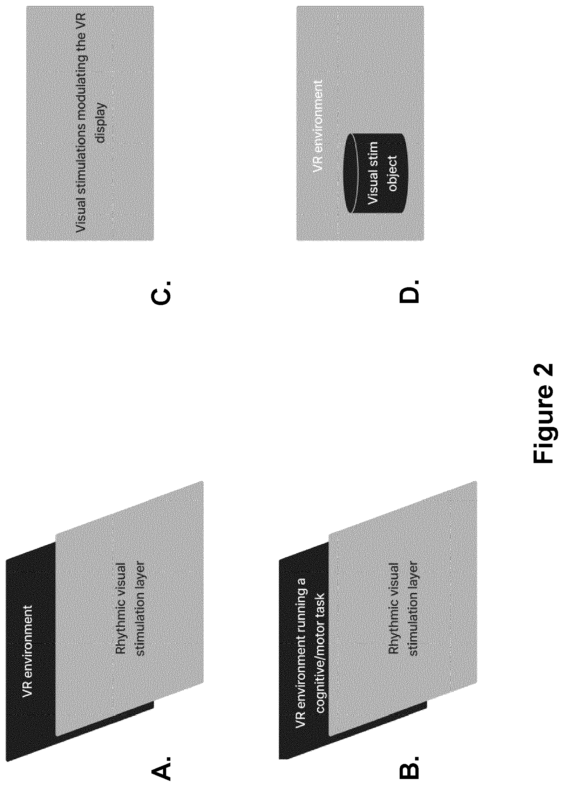

As indicated above, the visual stimulations are delivered and displayed on the screen of the headset (Figure 2). This can be performed by any on the following methods:
- delivering the visual stimulation by overlapping a VR environment while the patient passively watch the VR environment.
- delivering the visual stimulation by overlapping a VR environment that runs a cognitive or motor task and while the patient performs the task.
- delivering the visual stimulations by modulating the luminance of the VR display.
- delivering the visual stimulations by modulating the appearance of an object incorporated into the VR environment.

The first two methods require that the screen displays two layers of images: one layer presenting the virtual reality scenario, and another layer displaying the visual stimuli.

It is also possible to combine these methods, but this is not preferred.

### Visual stimuli

In preferred embodiments, the visual stimuli consist of pure sinusoidal modulation (sine waves, SM) of luminance level. The luminance level is modulated from 0% luminance (full black) to 100 % luminance (preferably full white but not limited to this specific color) with a 50 % duty cycle. This 50% duty cycle means that for a 4Hz stimulations, the luminance level varies from 0% to 100% in 125 ms and to 100% to 0% in 125 ms. For 40Hz stimulations, a 50% duty cycle means that the luminance level varies from 0% to 100% in 12,5 ms and from 100% to 0% in 12,5 ms. These can be generated with any software available in the art.

One can also use isochrone modulation (square waves, IM) of on/off luminance level modulation. With isochrone modulation, the luminance level is modulated with a square wave and a 50% duty cycle, meaning that for a 4Hz stimulations, the luminance level is at 100% during 125 ms and at 0% during 125 ms. For a 40Hz stimulations, the luminance level is at 100% during 12,5 ms and at 0% during 12,5 ms.

For the 4Hz-40Hz combined stimulation, 40Hz stimuli are preferably nested at the peak of 4Hz frequencies with a 0° phase offset.

### Auditory stimuli

Appropriate auditory stimuli generally consist of amplitude modulated (AM) sounds. Amplitude modulation implies modulating a carrier frequency depending on the stimulation frequency. It is preferred to use carrier frequencies between 250Hz and 1000Hz with a 100 % modulation depth , which are inducing the most significant auditory evoked responses. Lower carrier frequencies are perceived better because they are related to speech perception. The sound volume may be set at 40-80db, for instance at 70 dB SPL , which is the optimal sound volume for sustainable auditory perception. For combined stimulation frequencies like 4Hz-40Hz, highest (40Hz) frequencies are preferably nested at the peak of lowest (4Hz) frequencies with a 0° phase offset.

Other methods can also induce auditory entrainment, such as frequency modulation (FM), amplitude modulation (AM), and isochrone modulation (IM) techniques. FM and AM methods are well-known for being used in radiocommunication to transmit information with a radio carrier frequency. They either modulate the frequency or the amplitude of the carrier frequency, generally from 250Hz to 10kHz. Isochrone modulation is an on/off auditory tone presented at the selected frequency. Methods can also adapt carrier frequencies and modulation depth.

### Multisensory audio-visual stimuli

Multisensory audio-visual stimuli are delivered by synchronizing the same stimuli used for visual and auditory stimulation respectively, at specific frequencies (e.g for Alzheimer's: 4Hz and 40Hz) either alone or combined. As indicated above, they are most preferably provided with a 0° phase offset between frequencies and stimuli sources, meaning a perfect synchronization (also called in-phase or phase-locked).

### Frequency of stimulation

Stimulations are modulated at specific frequencies which can range from 1 Hz to 100Hz. Preferred frequencies used for Alzheimer's or Mild Cognitive Impairments (MCI) are 4Hz alone, 40Hz alone and 4Hz and 40Hz combined. Depending on the patient, and on the recording of the EEG, it is possible to adapt the frequency, which can vary from 35Hz to 45 Hz. In auditory, visual and audiovisual stimulation settings.

### Neurostimulation

Electrodes can be implanted or present on the helmet engage neural targets (brain, spinal cord, peripheral nerves, etc.). They are connected to a power source that would deliver specific patterns of pulses. The stimulation patterns can be optimized according the data measured by the sensors to increase therapeutic effects (in particular the brain waves phases and/or amplitudes).

The invention also relates to a method for treating a patient, comprising performing a neurological stimulation with a system herein disclosed, so that the sensory stimulations to the patient improve the patient's condition or slow the worsening of the condition of the patient.

A typical session would consist in selecting a virtual reality scenario or series of virtual reality scenarios to be displayed during the session and a first sensory stimulation protocol on the control panel, and have these elements be displayed on the screen of the headset worn by the patient. This may be done by the patient, if autonomous enough, or by a caretaker. Generally, the first stimulation protocol would include performing visual stimulation at 4 Hz, 40 Hz or a combination of these types of stimulations. It is preferred when the first stimulation protocol also includes providing audio stimulation, preferably phase-locked with the visual stimulation.

The session would generally last from 30 minutes to about one hour (a 1-hour session is generally preferred), unless prematurely stopped in case of detection of epileptic pattern as disclosed above (or by the patient or the caretaker). The first virtual reality scenario and first sensory protocol would be amended if needed, upon analysis of the physiological parameters of the patient. As indicated above, one preferred way of adjusting the protocol would be to adjust the frequency of the visual stimulations (and of auditory stimulations) to the actual frequency of the brain waves as measured in real time from the patient to obtain synchronization of the stimuli and of the brain waves. It is generally preferred to measure both brain waves phases and amplitudes. The amplitude of the stimulations may also be adapted to the amplitude of the brain waves. In some embodiments, the intensity and/or the color of the visual stimuli is modified according to the amplitude of the brain waves (it can be decreased or increased, and the color may be changed to be a given color of the spectrum or by a mix of colors (such as white). The intensity (volume) and/or frequency (move to the high-pitched or low pitched sounds) of the sound (audio) stimuli may also be adjusted according to the amplitude of the brain waves, the goal being to provide stimuli that increase the amplitude of the waves.

Such sessions should preferably be repeated every day.

The patient is preferably a patient with a neurological disability, whether due to a chronic neurological disease or to an acute neurological disorder, such as stroke.

The disease may be include Stroke, spine injuries, epilepsy, sleep disorders, Alzheimer's Disease, Parkinson's Disease, Dementia with Lewy Bodies, Primary Progressive Aphasias (PPA), Frontotemporal Dementia, Corticobasal Syndrome, Progressive Supranuclear Palsy, and Posterior Cortical Atrophy, and others.

### FIGURES

Figure 1: Schematic view of a preferred system.
Figure 2: Schematic representation of methods for delivering visual stimulations through a VR display.

### DESCRIPTION OF THE SYSTEM

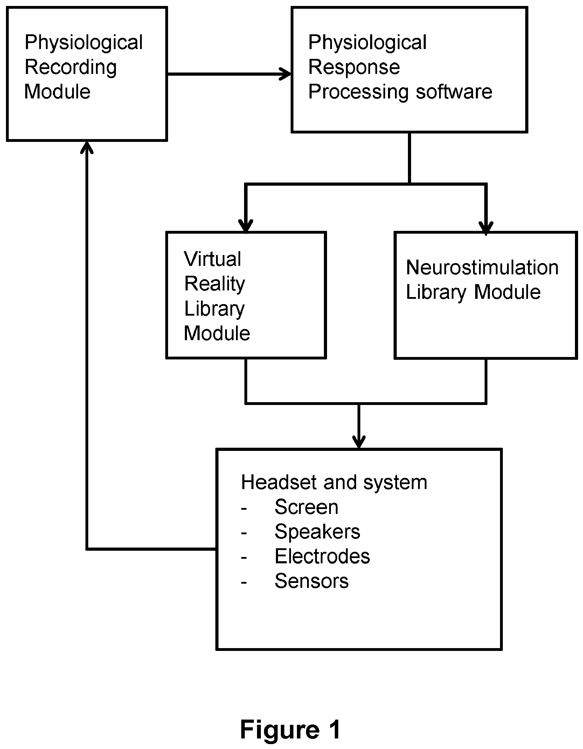

As depicted in Figure 1, the principle of operation of a system be comprise: a Virtual Reality scenario and a Neurostimulation (sensory) protocol are selected from the libraries of the Virtual Reality Library Module and Neurostimulation Library Module. They are transmitted for display to the system and headset that comprises sensors, measuring the physiological responses data. Such data is sent to the Physiological Recording Module for recording and analyzed in the Physiological Response Processing software (analysis and calculation module) which sends instruction to the Virtual Reality Library and Neurostimulation Library Modules for modifying the scenario and/or the neurostimulation. The Virtual Virtual Reality Module/Library contains scenarios for meditation, relaxation, breathing, virtual reality sceneries, cognitive tasks, motor tasks. The Neurostimulation Library Module / library contains protocols for visual stimulations, auditory stimulations, vagus nerve stimulations, transcranial direct current stimulations, transcranial alternating current stimulations, or other neurostimulations.

The system includes at least one virtual reality system, which includes a virtual reality headset which comprises a screen to be placed before the eyes of the patient, and speakers. The patient shall thus be exposed to virtual reality scenarios, on the screen of the helmet.

These scenarios are software associating visual universes, sound environments and can comprise tasks (cognitive or motor tasks) to be performed by the patient, requiring active participation of the patient during its virtual reality immersion. The scenarios can also comprise animated images, sounds, musics and the like. They are stored in at least a virtual reality module (or library) and are displayed on the screen through the speakers of the headset.

The system also comprises a control hardware, in which at least one library of virtual reality scenarios software, and at least one library of sensory stimulation software, are loaded. The control hardware can also contain a physiological response recording module, which stores in real time data from various parameters measured from the patient.

The library of sensory stimulation comprise instructions for providing visual stimulations (displayed on the screen of the headset), auditory stimulations, and optionally neurostimulations (such as vagus nerves stimulation, transcranial direct current stimulation (tDCS), transcranial alternative current stimulation (tACS) or other neurostimulations modules). As indicated, it is preferred to regularly provide the stimulations, that can thus be depicted as rhythmic stimulations.

The system also comprises sensors for measuring physiological parameters of the patient, that are stored in the physiological response recording module. These sensors are adapted to measure signals such as, signals from EEG (electroencephalogram), ECG (electrocardiogram), EOG (electrooculogram), PPG (photoplethysmogram), EMG (electromyogram), heart rate, respiratory rate, heart rate variability (HRV), pupillometry, electrodermal conductance and activity, accelerometer, photoplethysmogram (PPG), gyroscope and skin temperature, this list being not limitative. Suitable equipment (such as gloves, electrodes...) can be used to position the sensors on the appropriate location of the patient's body. The measured data (such as cardiac response, ocular response, skin response, blood volume change, muscular response) can be sent to the physiological response recording module, which stores the data from the various parameters in real time. The measured values may be sent to a physiological recording module that contains a CPU/GPU for processing these date, and can include an EEG amplifier, a transmission device (such as Bluetooth/Wifi antenna), and be autonomous (contains a power supply). Thes processor of the recording module can also send the measured parameters, on a regular basis, to a storage database, stored in a memory either remote or present in the module, to record the evolution of the parameters during the session, and overtime (variation of the parameters between two sessions).

The system may also comprise at least one analysis and calculation module comprising an algorithm loaded on the control hardware (physiological response processing software or module) and controlled by a processor. This module and algorithm is used to determine, in real time, the nature and variation of the physiological parameters measured and in particular determine whether the VR scenario or stimuli should be adapted.

Global health scores can be calculated using the analysis and calculation algorithm, based either on one physiological parameter or on several physiological parameters taken in combination. The values of the physiological parameters taken into account are measured in real time by the sensors and evaluated in real time by the calculation and analysis algorithm.

The calculation and analysis algorithm can also be used to measure an entrainment score, taking into account the data measured from the EEG of the patient (cerebral activity of the patient) during the session and in response to the various stimuli, or to the tasks asked in the virtual reality universe. Such EEG signal can also be compared to recorded patterns in order to identify any epileptic activity pattern and stop the session (in particular end the stimulations displayed to patient) in case of such activity.

The system can also include at least one display screen to show in real time the evolution of the entrainment score and/or of the global health score.

The display screen can also display the values measured in real time by the sensors, and the degree of progress of the session (virtual reality scenarios, sensory stimulations, duration of the session).

According to an embodiment, the display screen is integrated with the control hardware, which may be a computer, a cell phone or a touch-sensitive tablet.

Advantageously, the calculation algorithm is constantly improved by any artificial intelligence or "machine learning" system.

The invention also relates to a process that includes various successive execution steps, and feed-back loops to be adapted in real-time, as well as to a method for controlling the virtual reality headset and in particular the VR scenario and sensory protocols displayed thereon.

The method of treatment of a patient uses a virtual reality system to provide a virtual reality scenario to a patient an sensory stimulation, including visual stimulation on the screen displaying the virtual reality scenario.

The processes uses the data continuously measured in real time of at least one physiological parameter of the patient. Advantageously, it uses the data from multiple measured physiological parameters of the patient using one or more measurement sensors. Advantageously, the measured values are stored in a memory unit of the control hardware. Preferentially, the EEG of the patient is used as a measured physiological parameter.

The method makes use of at least one calculation and analysis algorithm to determine from the measured values of the physiological parameters, and in particular from the EEG data, whether the patient presents abnormal neural activity patterns (in particular epileptic patterns), and a controller to provide instructions to stop sending sensory stimulations and/or displaying virtual reality scenario to the patient. Preferably, the sensory stimulations protocol is immediately stopped and the display of the virtual reality scenario is also stopped although an "urgency" scenario, consisting in a scenario making it possible to gently put an end to the virtual reality scenario without bringing confusion to the patient. The processor controlling the calculation and analysis algorithm can thus send instructions to stop the display or to select new VR scenarios and/or stimulation protocols and send them to the headset.

In some embodiments, an algorithm is used to discard or correct measurements that are obviously erroneous or outside a range considered normal for a patient, so that they are not analyze by the algorithm of the calculation and analysis module..

The algorithm in the calculation and analysis module may be able to take various physiological parameters into consideration for determining the abnormal neural activity patterns.

The calculation and analysis algorithm shall also determine the response of the patient to the displayed virtual reality scenario and to the sensory stimulations, and send instruction to the databases of VR scenario and of sensory stimulations protocols to make those evolve depending on the patient's response.

Thus, upon receipt of the data from the sensory captors, the VR scenario and/or the sensory protocols can be changed and selected, in real time from their specific databases. The selection is then broadcasted into the virtual reality system headset worn by the patient, and in particular on the screen.

The method thus makes it possible to adapt or modulate in real time the selection of VR scenario and stimulation protocols. This adaptation or modulation is carried out according to the data from the measured parameters, as herein disclosed. Thus, depending on the response of the patient, the control/adapation/modulation process is repeated if necessary.

The software may thus integrate biofeedback or neurofeedback technique parameters to help optimize the stimulation protocols and VR scenarios.

The invention also relates to a non-transitory computer readable medium such as a memory, including computer executable instructions which when executed by one or more processors, such as the processor controlling the calculation and analysis algorithm cause the system herein disclosed to perform the steps of: selecting a VR scenario and/or a stimulation protocol and sending the selected event to a headset for display on the screen of the headset.

The advantages of the systems and processes herein disclosed include:
- Easy home-based access and performance
- easy patient's remote monitoring
- Facilitation of neurological disorders' diagnoses thereby reducing time and cost
- Facilitation of the delivery of noninvasive brain stimulations at home
- Increasing adherence with digital therapeutics by making it engaging, pleasant and funny
- Improving and accelerating the patient's path (slowing patient's health deterioration)
- Improving prevention, care quality, medical practices, personalization of medical guidelines based on actual daily health measurements
- Providing targeted and individualized power precision medicine solutions for neurological disorders, while making brain health solution more accessible
- Generally speaking, improving patient's and caregivers quality of life, while pushing back the need for advanced assistance
- Allowing medical teams to save time in monitoring patients

In summary, the invention provides a system for brain stimulation, comprising:
(a) at least one virtual reality system comprising a virtual reality headset, sensors and apparatus for continuously measuring in real time at least one physiological parameter of the patient, wherein the virtual reality headset comprises a screen
(b) at least one control hardware in which a first library of sensory stimulations protocols is loaded, and a second library of virtual reality scenarios is loaded
(c) at least one calculation and analysis algorithm loaded on the control hardware to determine the nature of the physiological parameter measured by the sensors apparatus devices, and to control the operation of the virtual reality system according to the nature of the measured physiological parameter by :
   i) selecting in real time from the first library a sensory stimulation protocol or a succession of sensory stimulation protocols, according to the nature of the measured physiological parameter, wherein the sensory stimulation protocol or succession of sensory stimulation protocols comprise visual, and preferably auditory (audio) sensory stimulation elements. These are provided at first specific frequencies, and a first specific intensities.
   ii) selecting in real time from the second library a virtual reality scenario or a succession of virtual reality scenarios, according to the nature of the measured physiological parameter; and
   iii) adapting or modulating in real time the sensory stimulation protocols and the virtual reality scenarios selected as a function of the evolution of the measured physiological parameter to provide the patient with personalized sensory stimulations (in particular by modifying the first frequencies to adjust such to the brain waves frequencies, and by also adjusting the intensity, audio frequencies, colors to increase the amplitude of the brain waves of the patient) able to optimally stimulate the brain activity of the patient.
wherein the system is configured to broadcast the selected virtual reality scenarios selection and the visual sensory stimulation elements on the screen of the virtual reality headset.

In the system, the first library preferably comprises protocols providing visual stimulations comprising light bursts at specific frequencies, preferably comprised between 1 Hz and 80 Hz.

In particular, the light bursts are presented at 4 Hz, 40 Hz and both 4 or 40 Hz.

It is preferred when the virtual reality headset further comprises integrated audio speakers for delivering auditory (audio, sound) stimulation protocols. These auditory stimuli are preferably delivered to the patient, being phase-locked with the visual stimulations. Generally speaking, is preferred when the system enables a perfect synchronization of auditory and visual stimuli, a phase-locking between stimulation frequencies (if multiple ones are combined e.g 4-40Hz coupling), and the adjustment/modification of the libraries is made so as to select the stimulation frequencies which are phase-locked with corresponding endogenous frequencies (brain waves frequencies).

In the most preferred embodiment, the measured physiological parameter of the patient comprises at least brain activity and the sensors comprise a ElectroEncephaloGraphy headset (EEG).

In particular, c) is performed by
a) analysis of brain oscillations during visual stimulation at a given frequency in the headset, from the EEG recording sent to the analysis and calculation module
b) quantification of the predominant brain frequencies
c) analysis of the amplitude and phase of the brain oscillations
d) adapting the onset and frequency of the stimuli (visual and optionally auditory stimulations, while maintaining phase synchronization between visual and auditory stimulations) for synchronization with the phase and amplitude of the measured target brain oscillations.

In preferred embodiment, the refreshing frequency of the screen is 120Hz or higher. In some embodiments, the measured physiological parameter of the patient comprises at least one of heart rate and body temperature. This makes it possible to establish health scores, and also to adapt the scenarios of the VR environment. In some embodiments, the virtual reality headset further comprises and an electrical stimulator for peripheral nerve stimuli. Generally, the scenarios of the second library comprises reproduction of places and spaces in order to allow the patient to practice relaxation exercises or improve cognitive abilities, and optionally comprise cognitive games to improve cognitive and/or functional abilities of the patient. The system further preferably comprises a user interface for selecting a stimulation protocol at the start of the session, and/or a software module for transmitting the physiological parameter data to a remote server via a computer network.

The invention also provides a computer-implemented method for controlling a virtual reality headset, comprising
a) Having a control hardware receive data measured from physiological parameters of a patient, wherein the patient wears the virtual reality headsets and sensors to measure the physiological parameters, and wherein a virtual reality scenario is displayed to the patient on the screen of the virtual reality headset, and sensory stimulations are applied to the patient wherein the sensory stimulations include visual stimulations displayed on the screen of the headset
b) Analyzing the nature of the data received in a), through a calculation and analysis algorithm implemented by a processor of the control hardware
c) Amending the sensory stimulations and/or the virtual reality scenario or selecting a new sensory stimulations protocol and/or a new virtual reality scenario according to the nature of the data analyzed in b)
d) Having the control hardware send instructions to the virtual reality headset for displaying the new or amended sensory stimulations protocol and/or virtual reality scenario.

As indicated above, it is of high interest when the physiological parameters comprise brain waves measured by electroencephalogram. The visual stimulations generally comprise emitting light bursts at specific frequencies, preferably comprised between 1 Hz and 80 Hz, in particular the light bursts are presented at 4 Hz, 40 Hz and both 4 or 40 Hz. In a preferred embodiment, the sensory stimulations further comprise auditory stimulations, preferably phase-locked with the visual stimulations.

It is preferred when b) comprises
i) analysis of brain oscillations during visual stimulation at a given frequency in the headset, from the EEG recording sent to the analysis and calculation module
ii) quantification of the predominant brain frequencies
iii) analysis of the amplitude and phase of the brain oscillations.

It is preferred when c) comprises adapting the onset and frequency of the stimuli (visual and optionally auditory stimulations, while maintaining phase synchronization between visual and auditory stimulations) for synchronization with the phase and amplitude of the measured target brain oscillations.

It is to be noted that the embodiments described above may be modified by the person skilled in the art, who may replace a technical feature by an equivalent technical feature, and a given execution step by an equivalent execution step, without leaving either the scope or the framework of the invention defined by the claims.

## Claims

1. A system for brain stimulation, comprising:
(a) at least one virtual reality system comprising a virtual reality headset, sensors and apparatus for continuously measuring in real time at least one physiological parameter of the patient, wherein the virtual reality headset comprises a screen
(b) at least one control hardware in which a first library of sensory stimulations protocols is loaded, and a second library of virtual reality scenarios is loaded
(c) at least one calculation and analysis algorithm loaded on the control hardware to determine the nature of the physiological parameter measured by the sensors apparatus devices, and to control the operation of the virtual reality system according to the nature of the measured physiological parameter by :
i) selecting in real time from the first library a sensory stimulation protocol or a succession of sensory stimulation protocols, according to the nature of the measured physiological parameter, wherein the sensory stimulation protocol or succession of sensory stimulation protocols comprise visual sensory stimulation elements
ii) selecting in real time from the second library a virtual reality scenario or a succession of virtual reality scenarios, according to the nature of the measured physiological parameter; and
iii) adapting or modulating in real time the sensory stimulation protocols and the virtual reality scenarios selected as a function of the evolution of the measured physiological parameter to provide the patient sensory stimulations able to stimulate the brain activity of the patient.
wherein the system is configured to broadcast the selected virtual reality scenarios selection and the visual sensory stimulation elements on the screen of the virtual reality headset.

2. The system of claim 1, wherein the first library comprises protocols providing visual stimulations comprising light bursts at specific frequencies, preferably comprised between 1 Hz and 80 Hz, in particular at 4 Hz, 40 Hz and both 4 or 40 Hz.

3. The system of any one of claims 1 or 2, wherein the virtual reality headset further comprises integrated audio speakers for delivering auditory stimuli.

4. The system of claim 3, wherein the auditory stimuli are phase-locked with the visual stimulations.

5. The system of any one of claims 1 to 4, wherein the physiological parameter of the patient comprises brain activity and the sensors comprise a ElectroEncephaloGraphy headset (EEG).

6. The system of claim 5, wherein c) is performed by
a) analysis of brain oscillations during visual stimulation at a given frequency in the headset, from the EEG recording sent to the analysis and calculation module
b) quantification of the predominant brain frequencies
c) analysis of the amplitude and phase of the brain oscillations
d) adapting the onset and frequency of the stimuli (visual and optionally auditory stimulations, while maintaining phase synchronization between visual and auditory stimulations) for synchronization with the phase and amplitude of the measured target brain oscillations.

7. The system of any one of claims 1 to 6, wherein the refreshing frequency of the screen is 120Hz or higher.

8. The system of any one of claims 1 to 7, comprising a user interface for selecting a stimulation protocol at the start of the session.

9. The system of any one of claims 1 to 8, further comprising a software module for transmitting the physiological parameter data to a remote server via a computer network.

10. A computer-implemented method for controlling a virtual reality headset, comprising
a) Having a control hardware receive data measured from physiological parameters of a patient, wherein the patient wears the virtual reality headsets and sensors to measure the physiological parameters, and wherein a virtual reality scenario is displayed to the patient on the screen of the virtual reality headset, and sensory stimulations are applied to the patient wherein the sensory stimulations include visual stimulations displayed on the screen of the headset
b) Analyzing the nature of the data received in a), through a calculation and analysis algorithm implemented by a processor of the control hardware
c) Modifying or adjusting the sensory stimulations and/or the virtual reality scenario or selecting a new sensory stimulations protocol and/or a new virtual reality scenario according to the nature of the data analyzed in b)
d) Having the control hardware send instructions to the virtual reality headset for displaying the new or amended sensory stimulations protocol and/or virtual reality scenario.

11. The method of claim 10, wherein the physiological parameters comprise brain waves as measured by electroencephalogram.

12. The method of claim 10 or 11, wherein the visual stimulations comprise emitting light bursts at specific frequencies, preferably comprised between 1 Hz and 80 Hz, in particular at 4 Hz, 40 Hz and both 4 or 40 Hz.

13. The method of any one of claims 10 to 12, wherein the sensory stimulations further comprise auditory stimulations, preferably phase-locked with the visual stimulations.

14. The method of any one of claims 10 to 13, wherein b) comprises
i) analysis of brain oscillations during visual stimulation at a given frequency in the headset, from the EEG recording sent to the analysis and calculation module
ii) quantification of the predominant brain frequencies
iii) analysis of the amplitude and phase of the brain oscillations.

15. The method of claim 10 to 14, wherein c) comprises adapting the onset and frequency of the stimuli (visual and optionally auditory stimulations, while maintaining phase synchronization between visual and auditory stimulations) for synchronization with the phase and amplitude of the measured target brain oscillations.
